# EUROPEAN PATENT APPLICATION

(11) **EP 4 736 937 A1**
(43) Date of publication of application: **06.05.2026**
(21) Application number: 25212579.4
(22) Date of filing: 31.10.2025
(51) Int. Cl.: A61N 2/00

(54) **MAGNETOTHERAPY SUPPORT DEVICE**

(30) Priority: 04.11.2024 IT 202400024612
(71) Applicant: Tenortho S.R.L., 20853 Biassono (Monza Brianza) (IT)
(72) Inventor: TENTORIO, Alessio, 20853 BIASSONO (MONZA BRIANZA) (IT)
(74) Representative: Zermani Biondi Orsi, Umberto

(57) **Abstract**

Magnetotherapy support device (1) comprising a main body (100, 200, 300, 400) made of flexible material to wrap itself at least partially around a joint to be treated and switchable into at least two configurations corresponding to two different joints to be treated. The support device (1) comprises at least one generator device (G) generating an electromagnetic field which can be associated with the main body (100, 200, 300, 400).

## Description

The present invention relates to a magnetotherapy support device. In other words, the present invention is part of the technical field of medical devices for the treatment of muscle and joint pain.

To perform a magnetotherapy treatment, as is known, use is made of support bodies applicable to different regions of the body and of generator devices generating an electromagnetic field and being applied or applicable to each support body.

The known support bodies are only applicable to a single joint, e.g. nowadays, different support bodies are used to perform a magnetotherapy treatment on the knee joint and the elbow joint: one shaped to be wrapped around the patient's leg and the other shaped to be wrapped around the patient's arm.

The known support bodies therefore suffer from the drawback of not being versatile and therefore of making it necessary to use different support bodies for each joint to be treated.

The production of the support bodies therefore requires as many production lines as there are joints of interest and is therefore expensive. Moreover, in this situation it is necessary to prepare a large number of support bodies in the warehouse, equal to the number of areas/joints to be treated, with the consequent drawbacks in terms of practicality and volumes of containment of the supports themselves.

The known support bodies allow extremely limited adaptability to the joints, especially if positioned at an angle of less than 180 degrees. The known support bodies therefore suffer from the drawback of not being usable during the common movements of a patient, forcing the latter to remain lying or sitting, even in a medical cabinet, throughout the treatment.

With reference to the generator device generating a magnetic field, it is generally connectable to one or more predetermined portions of the support body, disadvantageously rendering the known magnetotherapy support devices inflexible. In other words, in the known magnetotherapy support devices the generator device generating a magnetic field is positionable in limited and predefined portions of the support body.

The technical task of the present invention is therefore to make available a magnetotherapy support device that is able to overcome the drawbacks arising from the prior art.

An object of the present invention is to make available a flexible and versatile magnetotherapy support device.

An object of the present invention is to make available a magnetotherapy support device having reduced production costs.

The technical task mentioned and the objects stated are substantially achieved by a magnetotherapy support device comprising the technical features set out in one or more of the appended claims. The dependent claims correspond to possible embodiments of the invention.

In particular, the support device comprises a main body made of flexible material to wrap itself at least partially around a joint to be treated.

The main body is switchable into at least two configurations corresponding to two different joints to be treated.

Advantageously, the main body makes the support device versatile and reduces its production costs since it can be used on different joints.

The support device comprises at least one generator device generating a magnetic field, which can be associated with the main body.

The main body and the generator device have reversible fastening means. The reversible fastening means are configured to stably engage the main body on a respective joint to be treated.

The reversible fastening means is configured to stably engage the generator device to a region of the main body.

The main body comprises an inner surface, intended to come into contact with the joint to be treated, and an outer surface opposite the inner surface.

Preferably, the fastening means extend along the entire outer surface.

Advantageously such an extension makes the main body versatile, since it is possible to engage the generator device generating a magnetic field in any portion of the outer surface of the main body. It follows that any region of the portion of the body to which the main body is wrapped can be treated.

Further characteristics and advantages of the present invention will become clearer from the indicative, and therefore non-limiting, description of an embodiment of a magnetotherapy support kit.

Such description will be set forth herein below with reference to the accompanying drawings, provided for merely indicative and therefore non-limiting purposes, wherein:
- figure 1 shows a schematic view of the device in a first embodiment;
- figures 2-7 show schematic views of the device of figure 1 in different use configurations;
- figure 8 shows a schematic view of the device in a second embodiment;
- figures 9, 10 and 18 show schematic views of the device of figure 2 in different use configurations;
- figure 11 shows a schematic view of the device in a third embodiment;
- figures 12-14 show schematic views of the device of figure 11 in different use configurations;

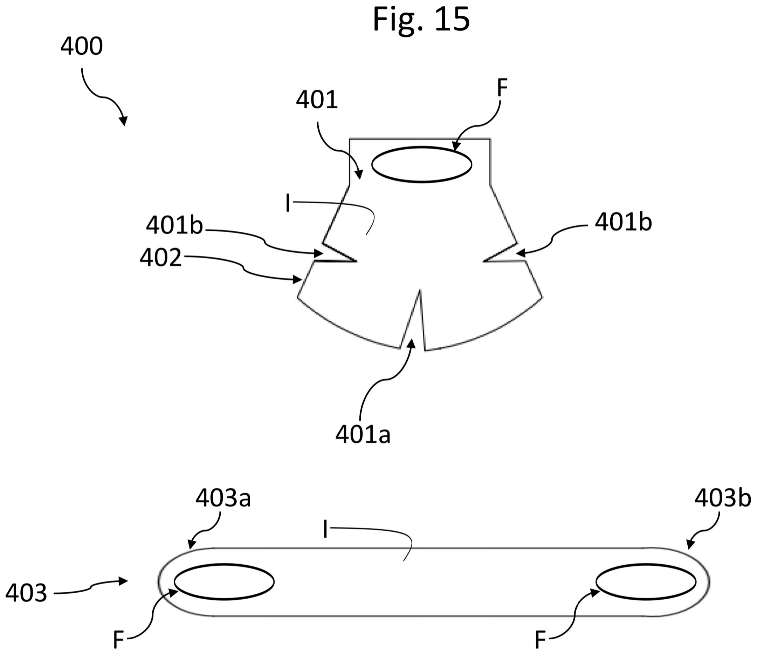

- figure 15 shows a schematic view of the device in a fourth embodiment;

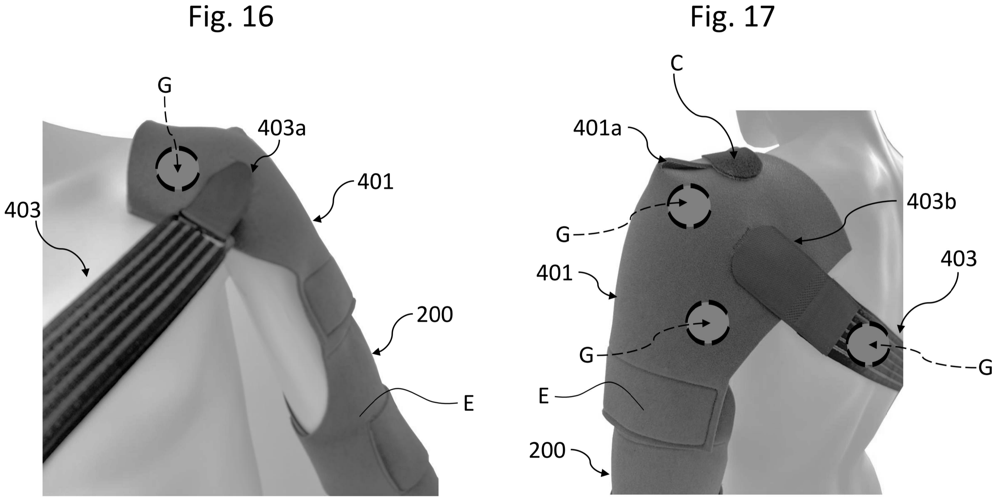

- figures 16 and 17 show schematic views of the device of figure 4 in a use configuration.

Within the scope of the present description, a magnetotherapy support device as a whole has been indicated with reference numeral 1, which will be referred to as "device 1" for simplicity in the following of the present description.

The device 1 comprises a main body 100, 200, 300, 400 adapted to be wrapped at least partially around a joint to be treated.

The main body 100, 200, 300, 400 is made of flexible, preferably elastic material.

This type of material allows an easy wrapping of the main body 100, 200, 300, 400 around the joint to be treated.

The main body 100, 200, 300, 400 is switchable into at least two configurations corresponding to two different joints to be treated.

In other words, the main body 100, 200, 300, 400 is switchable at least between a first configuration in which it is at least partially wrapped around a first joint to be treated and a second configuration in which it is at least partially wrapped around a second joint to be treated different from the first joint to be treated.

As will be clearer from the following of the present description, the main body 100, 200, 300, 400 is therefore orientable and positionable in space in different operating configurations depending on the joint to be treated. The main body 100, 200, 300, 400 is advantageously versatile: it is possible to apply a single main body 100, 200, 300, 400 to different joints according to needs. The production and storage costs of the main body 100, 200, 300, 400 are therefore reduced: the main body 100, 200, 300, 400 is adaptable to different joints and does not require as many production lines, nor as many warehouse areas, as there are joints of interest.

The device comprises at least one generator device "G" generating an electromagnetic field which can be associated with the main body 100, 200, 300, 400.

The main body 100, 200, 300, 400 and the generator device "G" have reversible fastening means "F" configured to stably engage the generator device "G" to a region of the main body 100, 200, 300, 400.

The reversible fastening means "F" are also configured to stably engage the main body 100, 200, 300, 400 on a respective joint to be treated.

In other words, a first and a second region of the main body 100, 200, 300, 400 are reversibly engageable with each other to stably wrap the main body 100, 200, 300, 400 to the joint to be treated.

Preferably, the fastening means "F" are applied to an inner surface "I", intended to come at least partially into contact with the joint to be treated, and to an outer surface "E", opposite the inner surface "I", of the main body 100, 200, 300, 400.

Preferably, the main body 100, 200, 300, 400 has a plate-like shape.

The term "plate-like shape" means that the thickness of the main body 100, 200, 300, 400 is lower than 30%, preferably 15%, of an extension of its outer and inner surfaces.

Preferably, the fastening means "F" comprise a Velcro^{®}.

Preferably, the fastening means "F" extend over the entire outer surface "E". Advantageously, this extension of the fastening means "F" allows the generator device "G" to be applied to any region of the main body 100, 200, 300, 400. It follows that the device 1 is flexible since it allows treating by magnetotherapy any area of the joint to be treated wrapped by the main body 100, 200, 300, 400.

The main body 100, 200, 300, 400 can be made in different embodiments, in which it has different shapes. Each shape is functional to the adaptability of the main body 100, 200, 300, 400 to one or more joints.

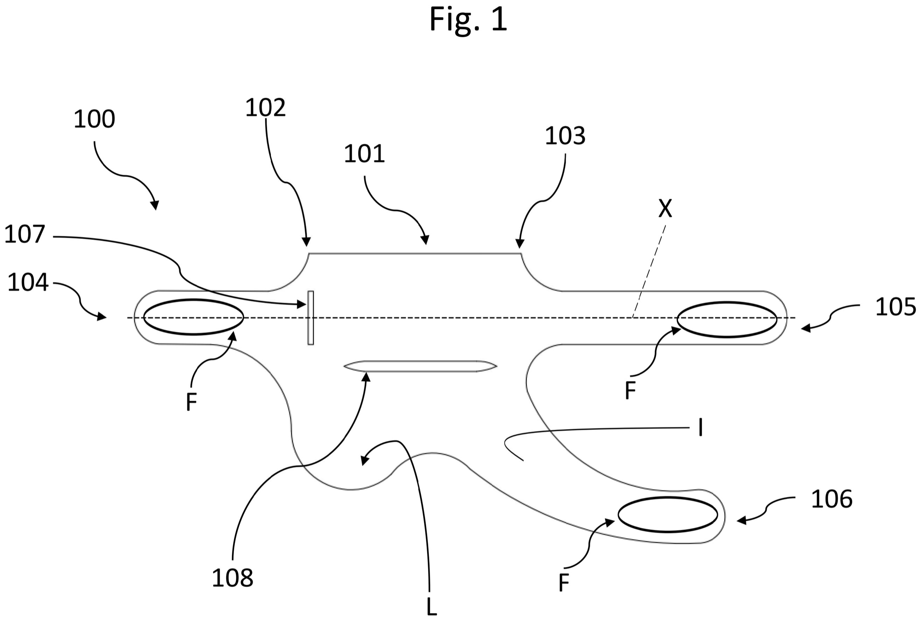

In accordance with a first embodiment (illustrated in figures 1 to 7) the main body 100 is switchable between a first, a second and a third configuration.

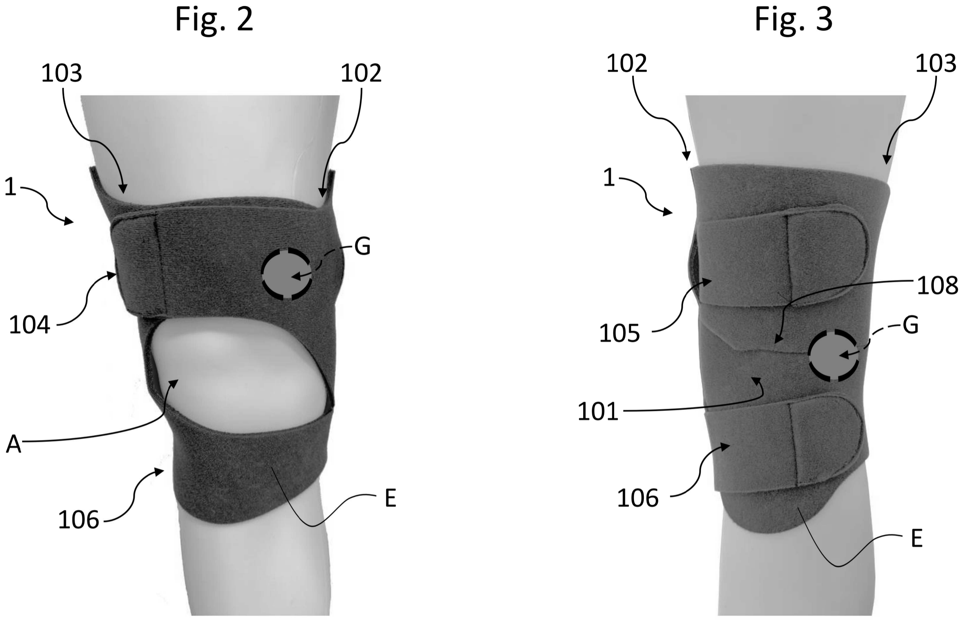

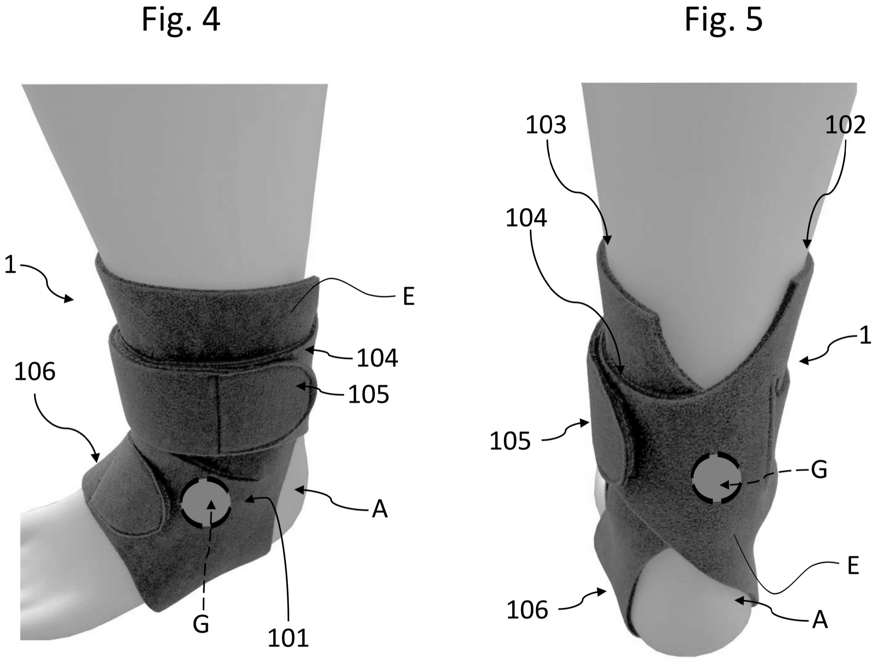

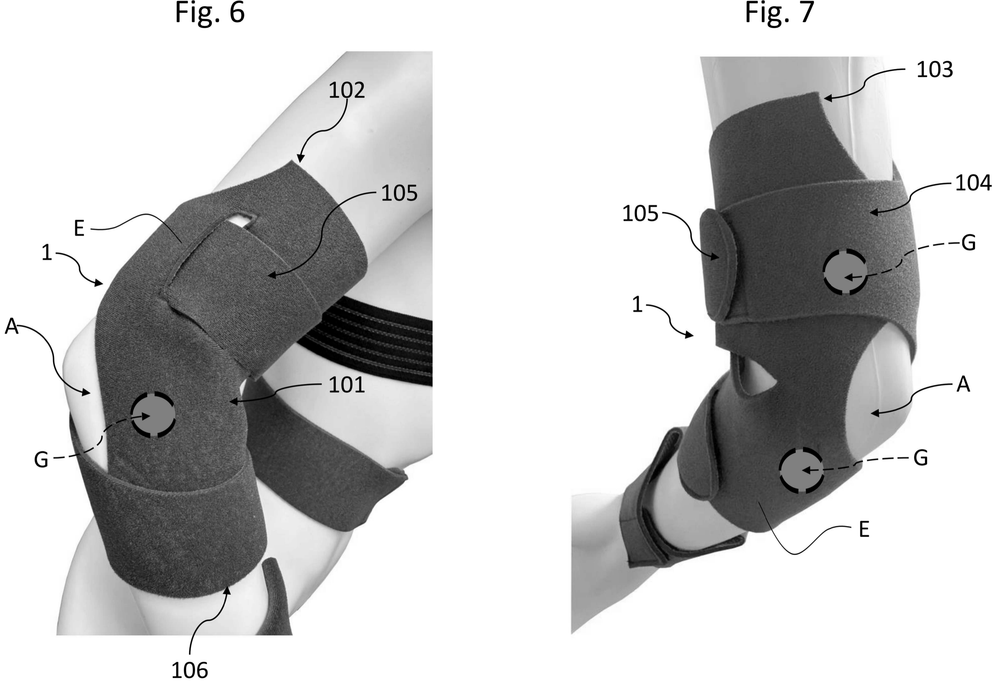

In the first configuration (figures 2 and 3), the main body 100 is wrapped around the knee joint, in the second configuration (figures 4 and 5) the main body 100 is wrapped around the ankle joint, in the third configuration (figures 6 and 7) the main body 100 is wrapped around the elbow joint. Structurally, with reference to figure 1, the main body 100 comprises a base 101 extending between a first end portion 102 and a second end portion 103 and a plurality of appendages 104, 105, 106 extending away from the base 101.

A first appendage 104 of the main body 100 extends away from the first end portion 102, preferably along a reference direction "X" transverse to the first 102 and the second end portion 103.

Preferably, the first appendage 104 has a linear shape.

Preferably, the first appendage 104 has a dimension along the reference direction "X" equal to at least half of the dimension of the base 101 along the reference direction "X". This dimension allows the first appendage 104 to be stably engaged with the base 101 and/or with the second appendage 105.

A second appendage 105 of the main body 100 extends away from the second end portion 103, preferably along the reference direction "X". Preferably, the second appendage 105 has a linear shape.

Preferably, the second appendage 105 has a dimension along the reference direction "X" equal to at least half of the dimension of the base 101 along the reference direction "X". This dimension allows the second appendage 105 to be stably engaged with the base 101 and/or with the first appendage 104 in a stable manner.

Preferably, the first 104 and the second appendage 105 are aligned with each other.

Preferably, the second appendage 105 has a dimension, measured along the reference direction "X" greater than the dimension of the first appendage 104 measured along the reference direction "X". These dimensions allow to engage the main body 100 with a joint to be treated in a stable manner: the second appendage 105 can be wrapped around the joint to be treated from a front portion thereof to a rear portion thereof (which are opposite with reference to a frontal plane).

A third appendage 106 of the main body 100 extends away from the second end portion 103 and is placed side by side to the second appendage 105 along the second end portion 103.

Preferably, the third appendage 106 has a curved shape. As can be seen in figure 2, this curved shape allows, in the first configuration, to wrap the portion of the knee joint proximal to the foot in an improved way. Preferably, the third appendage 106 has a concave shape having its own concavity facing the second appendage 105.

Preferably, the third appendage 106 has a dimension along the reference direction "X" equal to at least half of the dimension of the base 101 along the reference direction "X". This dimension allows the third appendage 106 to be stably engaged with the base 101.

Preferably, the base 101 has a lobe "L" defining a portion of the first end portion 102. In other words, the lobe "L" is interposed between the first appendage 104 and the third appendage 106.

Preferably, the base 101 has a through opening 107 in the first end portion 102 into which the second appendage 105 is insertable. By inserting the second appendage 105 within the through opening 107 it is possible to position the first main body 100 in a use configuration in which it has a substantially annular portion (delimited by the base 101 and by the second appendage 105 which is inserted within the through opening 107) into which the joint to be treated is inserted.

Preferably, the base 101 has a through-notch 108 oriented transversely with respect to the first 102 and the second 103 end portion. As will be clearer from the continuation of the present description, the through-notch 108 allows greater mobility of the joint to be treated when the main body 100 is wrapped around the latter.

Preferably the fastening means "F" are arranged on the inner surface "I" of the main body 100 at least at end regions, distal with respect to the base 101, the first 104, the second 105 and the third 106 appendage.

Both in the first (figures 2 and 3), in the second (figures 4 and 5) and in the third (figures 6 and 7) configuration the second 105 and the third 106 appendage are engaged by the fastening means "F" to the base portion 101 and the first appendage 104 is engaged by the fastening means "F" to the second appendage 105.

In detail, the second appendage 105 is in contact with the first end portion 102 of the base 101 and is engaged by the fastening means "F" with the base 101.

The second appendage 105 wraps, in cooperation with the base 101, the thigh in the first configuration, a portion of the leg between the knee and the ankle in the second configuration and the arm in the third configuration.

Within the scope of the present description, "arm" means a portion of an upper limb between the shoulder and the elbow.

In the first configuration (figures 2 and 3), the first appendage 104 is engaged by the fastening means "F" with the second appendage 105 to wrap the thigh, in cooperation with the base 101.

In other words, in the first configuration the first 104 and the second 105 appendage define with the base portion 101 a closed profile within which, in the first configuration, the thigh is inserted.

In the second (figures 4 and 5) and third (figures 6 and 7) configuration, the first appendage 104 is in contact with the second end portion 103 of the base 101 and is engaged by the fastening means "F" with the base 101.

In the second (figures 4 and 5) and third (figures 6 and 7) configuration, the second appendage 105 is engaged by the fastening means "F" with the first appendage 104 and, preferably, the first appendage 104 is at least partially interposed between the base 101 and the second appendage 105.

The first appendage 104 wraps, in cooperation with the base 101 and with the second appendage 105, a portion of the leg between the knee and the ankle in the second configuration, the arm in the third configuration.

In other words, in the second configuration the first 104 and the second 105 appendage define with the base portion 101 a closed profile within which, in the second configuration, a portion of the leg between the ankle and the knee is inserted, and within which, in the third configuration, the arm is inserted.

Both in the first (figures 2 and 3), in the second (figures 4 and 5) and in the third (figures 6 and 7) configuration, the third appendage 106 is engaged by the fastening means "F" at least with the first end portion 102 of the base 101.

The third appendage 106 wraps, in cooperation with the base 101, a portion of the leg between the knee and the ankle in the first configuration, a portion of the foot between the heel and the forefoot in the second configuration, the forearm in the third configuration.

Within the scope of the present description, "forearm" means a portion of the upper limb between the wrist and the elbow.

Preferably, both in the first (figures 2 and 3) and in the second (figures 4 and 5) and in the third (figures 6 and 7) configuration, the first 104, the second 105 and the third 106 appendage delimit an opening "A".

The opening "A" is configured to receive in insertion the patella in the first configuration, the heel in the second configuration, the elbow in the third configuration.

The lobe "L" in the first configuration (figures 2 and 3) is positioned at the rear of the knee and allows to wrap a portion of the leg below the knee (taking a direction going from the knee to the foot as a reference), thus allowing to apply the generator device "G" also in this portion of the leg. The through-notch 108 in the first configuration (figures 2 and 3) is positioned at the rear of the knee at the joint between the tibia and femur. Advantageously, the through-notch 108 allows the user to bend the knee joint at will and without discomfort, preventing folds from forming when the knee joint is flexed.

Both the through-notch 108 and the shape of the main body 100 determine a compatibility of the main body 100 with a mobility of the knee, ankle and elbow joints. In other words, the device 1 is usable by a user even during common movements of the knee, ankle and elbow joints. For example, the device 1 can be used by a user who is sitting, lying down, while walking, etc.

Both in the first (figures 2 and 3), in the second (figures 4 and 5), and in the third (figures 6 and 7) configuration the second appendage 105 is inserted within the through opening 107.

It is therefore possible to insert the second appendage 105 within the through opening 107 to define a closed profile into which a limb of interest can be inserted and, once inserted, it is possible to pull the second appendage 105 until the base 101 and the second appendage 105 adhere to the limb: by engaging the second appendage 105 with the base 101, the first appendage 104 with the second appendage 105 and the third appendage 106 with the base 101 by the fastening means "F", the main body 100 is stably engaged to the limb of interest.

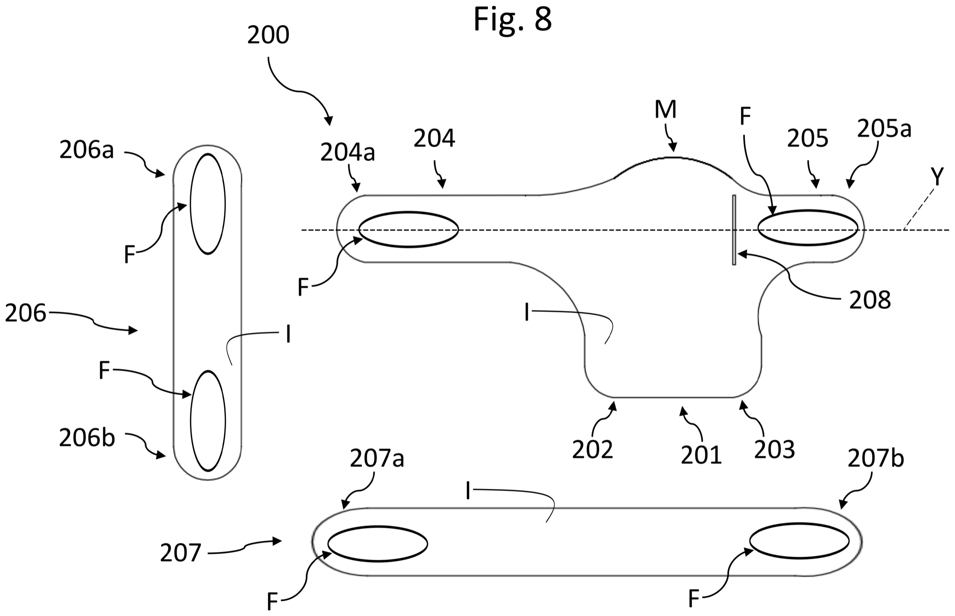

In accordance with a second embodiment (illustrated in figures 8 to 10 and 18), the main body 200 is switchable into a primary configuration and a secondary configuration.

In the primary configuration, the main body 200 is wrapped around the torso for contact with the lumbar portion of the spine, in the secondary configuration, the main body 200 is wrapped around the arm.

Structurally, the main body 200 comprises a main portion 201, which extends between a left edge 202 and a right edge 203 opposite to each other, and a plurality of bands 204, 205 extending away from the main portion 201.

A left band 204 and a right band 205 of the main body 200 extend away from the left edge 202 and the right edge 203 of the main portion 201, respectively.

Preferably the left band 204 extends along a reference direction "Y" transverse to the left edge 202 and right edge 203; preferably the left band 204 has a linear shape.

Preferably the right band 205 extends along the reference direction "Y"; preferably the right band 205 has a linear shape.

Preferably, the right band 205 is aligned with the left band 204. Preferably, at least one of the left band 204 and the right band 205 has a dimension along a reference direction "Y" equal to at least half of a dimension of the main portion 201 along the reference direction "Y". Preferably, the left band 204 has a dimension, measured along the reference direction "Y" greater than a dimension of the right band 205 measured along the reference direction "Y". These dimensions allow the main body 200 to be engaged with the arm in the secondary configuration in a stable manner: the left band 204 can be wrapped around the arm from a front portion thereof to a rear portion thereof (which are opposite with reference to a front plane).

Preferably, the main portion 201 has a through opening 208 passing through the right edge 203 and within which the left band 204 is insertable. The main body 200 comprises a first auxiliary band 206 reversibly engageable by the fastening means "F" to the left 204 and right 205 bands. In other words, an end 204a of the left band 204 is removably engageable with an end 206a of the first auxiliary band 206 and an end 205a of the right band 205 is removably engageable with an end 206b of the first auxiliary band 206.

The main body 200 comprises a second auxiliary band 207 reversibly engageable by the fastening means "F" to the main portion 201. For example, one end 207a of the second auxiliary band 207 is removably engageable with the left edge 202 of the main portion 201 and one end 207b of the second auxiliary band 207 is removably engageable with the right edge 203 of the main portion 201.

Preferably, the fastening means "F" are arranged on portions of the inner surface "I" of the main body 200 defining the ends 204a of the left band 204, 205a of the right band 205, 206a and 206b of the first auxiliary band 206, 207a and 207b of the second auxiliary band 207.

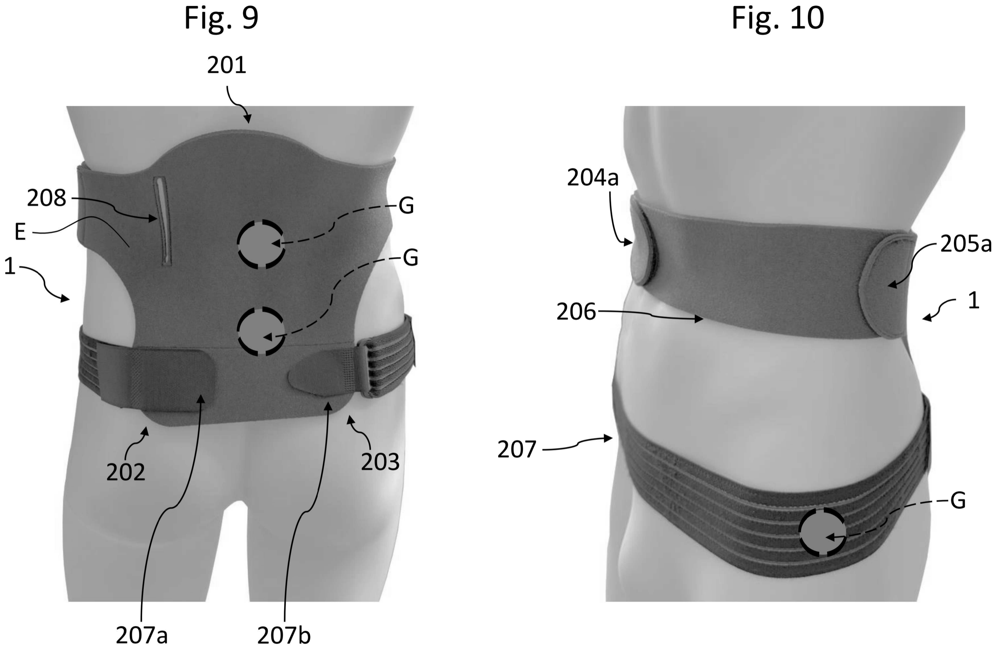

In the primary configuration (figures 9 and 10), the left band 204 and the right band 205 are engaged by the fastening means "F" with respective ends 206a, 206b of the first auxiliary band 206.

In the primary configuration (figures 9 and 10), the left 204 and right 205 bands in cooperation with the main portion 201 and the first auxiliary band 206 wrap around the torso, preferably near the navel.

In the primary configuration (figures 9 and 10), the second auxiliary band 207 has its own ends 207a, 207b engaged by the fastening means "F" to the left edge 202 and to the right edge 203 of the main portion 201.

In the primary configuration (figures 9 and 10), the second auxiliary band 207 wraps, in cooperation with the main portion 201, the torso.

Preferably, in the primary configuration (figures 9 and 10) the main portion 201 is arranged in contact with the lumbar portion of the spine and the second auxiliary band 207 maintains the main portion 201 taut on the lumbar portion of the spine.

Preferably, a portion of the second auxiliary band 207 is in contact with the hip.

Thanks to the main body 200, it is therefore possible to treat by magnetotherapy the lumbar portion of the spine by engaging the generator device "G" with the main portion 201 and the hip by engaging the generator device "G" with the second auxiliary band 207. Alternatively, the main portion 201 may be arranged in contact with the hip joint and the second auxiliary band 207 maintains the main portion 201 taut on the hip joint.

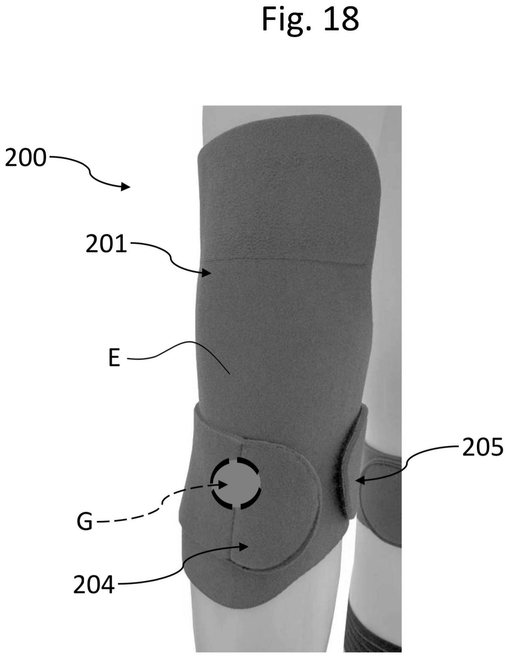

In the secondary configuration (figure 18) the left band 204 is in contact with the right edge 203 and is engaged by the fastening means "F" with the main portion 201 and the right band 205 is in contact with the left edge 202 and is engaged by the fastening means "F" with the main portion 201. In the secondary configuration (figure 18), the left band 204 and the right band 205, in cooperation with the main portion 201, wrap around the arm and, preferably, are in contact with the elbow.

Preferably, the main portion 201 comprises a lobe "M" interposed between the right band 205 and the left band 204. The lobe "M" in the auxiliary configuration allows to wrap a portion of the forearm proximal to the elbow. In order to place the main body 200 in the secondary configuration it is possible to wrap the arm by inserting the left band 204 within the through opening 208: by pulling the left band 204 until the left band 204 and the main portion 201 come into contact with the arm and subsequently by engaging the left band 204 and the right band 205 with the base portion 201, the main body 200 is stably engaged with the arm. In other words, in the auxiliary configuration the left band 204 is inserted within the through opening 208.

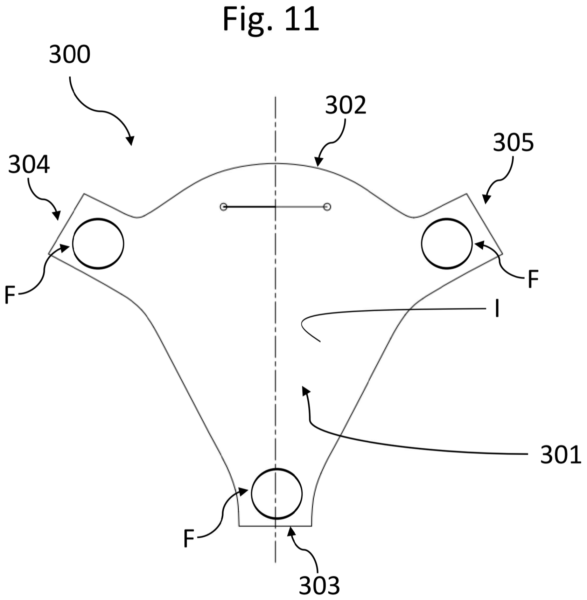

In accordance with a third embodiment, illustrated in figures 11-13, the main body 300 is switchable into a wrapping configuration in which it is wrapped around the wrist.

Structurally, the main body 300 comprises a cover portion 301 delimited by an upper end portion 302 and a lower end portion 303 opposite to each other.

Preferably, the main body 300 has a triangular shape: the upper end portion 302 is defined by one side of the triangular shape and the lower end portion 303 is defined by a vertex of the triangular shape opposite to said one side.

The main body comprises a first 304 and a second 305 fastening portion extending away from the upper end portion 302 and placed side by side along the upper end portion 302.

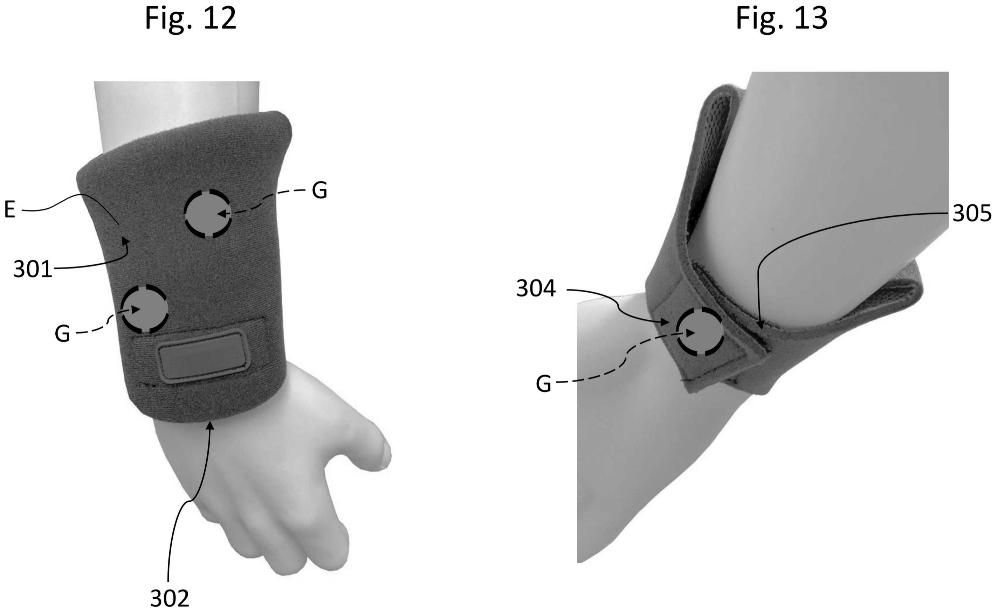

In the wrapping configuration (figures 12 and 13), the lower end portion 303 is in contact with the upper end portion 302; the first 304 and the second 305 fastening portions are engaged with each other by the fastening means "F" to wrap the cover portion 301 around the wrist. Preferably, the fastening means "F" are arranged in regions of the inner surface "I" of the main body 300 defining the lower end 303, the first 304 and the second 305 fastening portion.

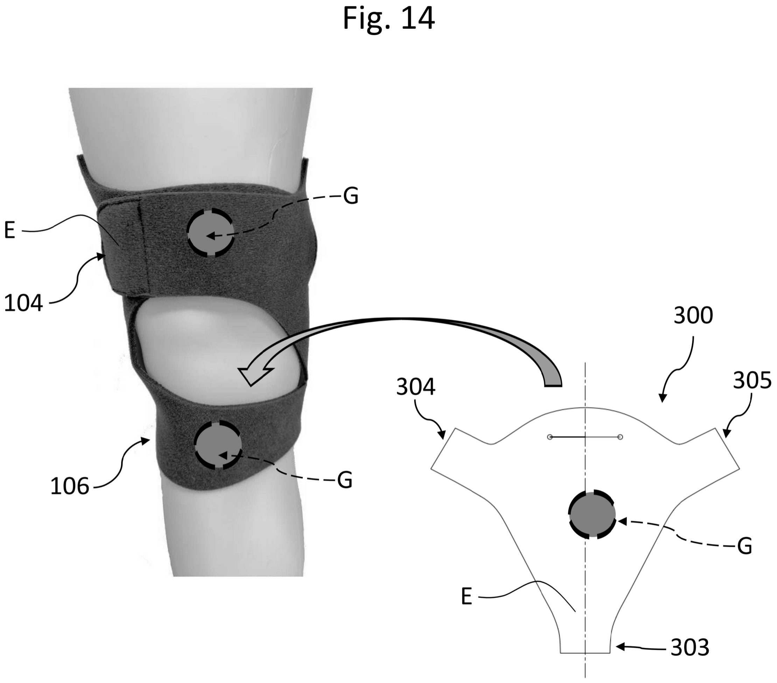

In accordance with one aspect of the present invention and with reference to figure 14:
- the lower end portion 303 of the main body 300 is engageable by the fastening means "F" with the third appendage 106 of the main body 100 in accordance with the first embodiment and
- the first 304 and the second 305 fastening portions can be engaged by the fastening means "F" with at least one of the first 104 and the second 105 appendage of the main body 100 in accordance with the first embodiment.

Therefore, the cover portion 301 is arranged to cover the opening "A".

In other words, the main body 300 in accordance with the third embodiment is switchable into a cover configuration in which it is engaged by the fastening means "F" with the main body 100 in accordance with the first embodiment.

The main body 300 in the cover configuration covers the patella when the main body 100 is in the first configuration, the heel when the main body 100 is in the second configuration, the elbow when the main body 100 is in the third configuration.

Advantageously, it is therefore possible to treat the region of the patella, heel or elbow by magnetotherapy by positioning the generator device "G" on the cover portion 301 when the main body 300 is in the cover configuration.

In accordance with a fourth embodiment, the main body 400 is wrappable around the shoulder and is switchable between a plurality of operating configurations wherein it is in contact with the shoulder and different portions of the cervical and/or thoracic spine. In other words, the main body 400 in each operating configuration is in contact with a different joint between thoracic or cervical vertebrae.

Structurally, the main body 400 comprises a wrapping body 401, delimited by a perimeter edge 402, and an auxiliary band 403, reversibly connectable by the fastening means "F" to the perimeter edge 402.

The wrapping body 401 is configured to be positioned in contact with the shoulder in each of the operating configurations and the auxiliary band 403 is configured to be positioned in contact with a different joint between cervical/thoracic vertebrae in each of the operating configurations. Preferably, the wrapping body 401 has a concave portion 401a configured to be positioned at the clavicle in each of the operating configurations. Preferably, the wrapping body 401 comprises a connection element "C" reversibly engageable by the fastening means "F" with the wrapping body 401 and which can be engaged with the margins delimiting the concave portion 401a to join them in one or more of the operating configurations. Preferably, the wrapping body 400 has auxiliary concave portions 401b opposite to each other and configured to be positioned in contact with a front portion and a rear portion (which are opposite to a front plane) of the shoulder.

Preferably the fastening means "F" are arranged on the inner surface "I" of the auxiliary band 403 at its ends 403a, 403b: in each operating configuration the ends 403a, 403b are engaged by the fastening means "F" to portions of the outer surface "E" of the wrapping body 401 proximal to the perimeter edge 402.

The auxiliary band 403 wraps around the torso in each of the operating configurations and is in contact with different joints of the cervical and/or thoracic spine in different operating configurations. By selecting the region of the outer surface "E" of the wrapping body 401 it is possible to select the joint between vertebrae of the thoracic/cervical spine with which the auxiliary band 403 is in contact.

It is therefore possible to treat several joints of the cervical/thoracic spine by magnetotherapy by applying the generator device "G" to the auxiliary band 403 and the shoulder joint by applying the generator device "G" to the wrapping body 401.

In accordance with an aspect of the present invention, a portion of the perimeter edge 402 opposite the concave portion 401a of the wrapping body 401 is engageable by the fastening means "F" with a perimeter portion, preferably opposite to the lobe "M" of the main portion 201 of the main body 200 in accordance with the second embodiment.

Preferably, the fastening means "F" are arranged on a portion of the inner surface "I" of the wrapping body 401 proximal to the portion of the perimeter edge 402 opposite to the concave portion 401a.

It is therefore possible to treat by magnetotherapy both the shoulder, by applying the generator device "G" to the wrapping body 401 and the portion of the arm adjacent to the shoulder by applying the generator device "G" to the main portion 201.

The present invention also makes available a kit comprising a first and a second support device 1.

Advantageously, the present invention is able to overcome the drawbacks arising from the prior art.

The fastening bodies 100, 200, 300, 400 are adaptable to different joints and are therefore flexible.

The fastening bodies when wrapped around a joint to be treated allow for an unimpeded movement thereof and are therefore wearable during the day.

## Claims

1. Magnetotherapy support device (1) **characterised in that** it comprises:
- a main body (100, 200, 300, 400) made of flexible material to wrap itself at least partially around a joint to be treated; said main body (100, 200, 300, 400) being switchable into at least two configurations corresponding to two different joints to be treated;
- at least one generator device (G) generating an electromagnetic field which can be associated with said main body (100, 200, 300, 400);
said main body (100, 200, 300, 400) and said generator device (G) having reversible fastening means (F) configured to stably engage the main body (100, 200, 300, 400) to a respective joint to be treated, and to stably engage the generator device (G) to a region of said main body (100, 200, 300, 400).

2. Support device (1) according to claim 1, wherein said main body (100) is switchable between a first configuration in which it is wrapped around the knee joint, a second configuration in which it is wrapped around the ankle joint, and a third configuration in which it is wrapped around the elbow joint.

3. Support device (1) according to claim 2, wherein the main body (100) comprises:
- a base (101) extending between a first end portion (102) and a second end portion (103) opposite to each other;
- a first appendage (104) extending away from the first end portion (102) of the base (101);
- a second appendage (105) extending away from the second end portion (103) and that is aligned with the first appendage (104);
- a third appendage (106) placed side by side to the second appendage (105) along the second end portion (103) of the base (101) and extending away from said second end portion (103),
wherein each of said first (104), second (105) and third (106) appendages has a dimension along a reference direction (X) transverse to the first (102) and second (103) end portions of said base (101) equal to at least half of a dimension of said base (101) along said reference direction (X), preferably the first (104) and second (105) appendages having a linear shape and the third appendage (106) having a curved shape facing the second appendage (105).

4. Support device (1) according to claim 3, wherein the base (101) has:
- an opening (107) passing through the first end portion (102) of said base (101) and wherein the second appendage (105) is insertable within said through-opening (107) in at least one of said first, second and third configurations; and/or
- a notch (108) passing through said base (101) and oriented transversely with respect to the first (102) and second (103) end portions of the base (101), wherein said through-notch (108) is positioned at the rear of the patella in the first configuration and at the front of the elbow in the third configuration in relation to a frontal plane.

5. Support device according to claim 3 or 4 wherein:
- the second appendage (105) in each of said first, second and third configurations is in contact with the first end portion (102) of the base (101) and is engaged by said fastening means (F) with said base (101) to wrap, in cooperation with the base (101), the thigh in the first configuration, a portion of the leg between the knee and the ankle in the second configuration, the arm in the third configuration;
- the first appendage (104) in the first configuration is engaged by said fastening means (F) with the second appendage (105) to wrap the thigh in cooperation with the base (101);
- the first appendage (104) in the second and third configurations is in contact with the second end portion (103) of the base (101) and is engaged by said fastening means (F) with the base (101) and with the second appendage (105) to wrap in cooperation with the base (101) a portion of the leg between the knee and the ankle in the second configuration, the arm in the third configuration; wherein preferably in the second and third configurations the first appendage (104) is at least partially interposed between the base (101) and the second appendage (105);
- the third appendage (105) is engaged by said fastening means (F) with at least the first end portion (102) of the base (101) to wrap in cooperation with the base (101) a portion of the leg between the knee and the ankle in the first configuration, a portion of the foot between the heel and the forefoot in the second configuration, the forearm in the third configuration; wherein the first (104), second (105) and third (106) appendages delimit an opening (A) configured to receive in insertion the patella in the first configuration, the heel in the second configuration, the elbow in the third configuration.

6. Support device (1) according to claim 1 wherein said main body (200) is switchable between a primary configuration in which it is wrapped around the torso and in contact with the lumbar portion of the spine, and a secondary configuration in which it is wrapped around the arm.

7. Support device (1) according to claim 6, wherein said main body (200) comprises:
- a main portion (201) extending between a left edge (202) and a right edge (203) opposite to each other;
- a left band (204) extending away from the left edge (202) of the main portion (201);
- a right band (205) extending away from the right edge (203) of the main portion (201) and aligned with the left band (204),
- a first auxiliary band (206) reversibly engageable by said fastening means (F) to the left (204) and right (205) bands;
- a second auxiliary band (207) reversibly engageable by said fastening means (F) to the main portion (201);
at least one of the left band (204) and the right band (205) having a dimension along a reference direction (Y) transverse to the left (202) and right (203) edge of the main portion (201) equal to at least half of a dimension of the main portion (201) along said reference direction (Y).

8. Support device (1) according to claim 7, wherein:
- in the primary configuration, the left band (204) and the right band (205) are engaged by said fastening means (F) with respective ends (206a, 206b) of the first auxiliary band (206) for wrapping the torso, and wherein the second auxiliary band (207) has its own ends (207a, 207b) engaged by said fastening means (F) to the left edge (202) and to the right edge (203) of the main portion (201) for wrapping the torso, in said primary configuration the main portion (201) being arranged in contact with the lumbar portion of the spine and preferably a portion of said second auxiliary band (207) being in contact with the hip;
- in the auxiliary configuration, the left band (204) is in contact with the right edge (203) and is engaged by said fastening means (F) with the main portion (201), and the right band (205) is in contact with the left edge (202) of the main portion (201) and is engaged by said fastening means (F) with the main portion (201); in said auxiliary configuration, the left band (204) and the right band (205) wrapping the arm in cooperation with the main portion (201);
preferably the main portion (201) having an opening (208) passing through the right edge (203) and within which the left band (204) is insertable when the main body (200) is in said secondary configuration.

9. Support device (1) according to claim 1 wherein said main body (300) is switchable to a wrapped configuration wherein it is wrapped around the wrist.

10. Support device (1) according to claim 9 wherein said main body (300) comprises
- a cover portion (301) delimited by an upper end portion (302) and a lower end portion (303) opposite to each other,
- a first (304) and a second (305) fastening portion extending away from the upper end portion (302) and placed side by side along said upper end portion (302),
wherein in the wrapped configuration the lower end portion (303) is in contact with the upper end portion (302) and the first and second fastening portions (304, 305) are engaged with each other by said fastening means (F) to wrap the cover portion (301) around the wrist.

11. Support device (1) according to claim 1 wherein said main body (400) is wrappable around the shoulder and is switchable between a plurality of operating configurations wherein it is in contact with the shoulder and different joints of the cervical and/or thoracic spine.

12. Support device (1) according to claim 11, wherein said main body (400) comprises:
- a wrapping body (401) configured to be positioned in contact with the shoulder in each of said operating configurations, wherein the wrapping body (401) is delimited by a perimeter edge (402) having a concave portion (402a) configured to be positioned at the clavicle in each of said operating configurations;
- an auxiliary band (402) reversibly connectable by said fastening means (F) to said perimeter edge (401), to wrap the torso in each of said operating configurations, the auxiliary band (402) being in contact with different portions of the cervical and/or thoracic spine in different operating configurations.

13. Support device (1) according to one or more of the preceding claims, wherein each main body (100, 200, 300, 400) is delimited by an outer surface (E) and an inner surface (I) and wherein said fastening means (F) extend throughout the outer surface (E) and at least part of the inner surface (I), preferably said fastening means (F) comprising a Velcro^{®}.

14. Kit comprising a first and a second support device (1) according to one or more of the preceding claims.

15. Support kit comprising:
- a first support device (1) according to one or more of claims 3-5 and a second support device (1) according to claim 10, wherein the lower end portion (303) of the main body (300) of the second support device (1) is engageable by said fastening means (F) with the third appendage (106) of the main body (100) of the first support device (1), and wherein the first (304) and second (305) fastening portions (F) of the main body (300) of the second support device (1) are engageable by said fastening means (F) with at least one of the first (104) and second (105) appendages of the main body (100) of the first support device (1);
and/or
- a first support device (1) according to claim 7 or 8 and a second support device (1) according to claim 12, wherein a portion of the perimeter edge (401) opposite said concave portion (401a) of the wrapping body (401) of the second support device (1) is engaged by the fastening means (F) with a perimeter portion of the main portion (201) of the main body (200) of the first support device (1).
